# EUROPEAN PATENT APPLICATION

(11) **EP 4 715 383 A1**
(43) Date of publication of application: **25.03.2026**
(21) Application number: 25159955.1
(22) Date of filing: 25.02.2025
(51) Int. Cl.: G01N 33/58, G01N 33/543, B01L 9/00, B01L 3/00

(54) **TEST STRIP FOR TESTING ANALYTE IN SAMPLE**

(30) Priority: 23.09.2024 CN 202411328176; 23.09.2024 CN 202411323820
(71) Applicant: Zhejiang Orient Gene Biotech Co., Ltd, Anji Huzhou Zhejiang 313300 (CN)
(72) Inventor: SHEN, Lili, Huzhou, 313300 (CN); TANG, Qili, Huzhou, 313300 (CN); FANG, Qingxian, Huzhou, 313300 (CN)
(74) Representative: Piotrowicz, Pawel Jan Andrzej

(57) **Abstract**

The invention discloses a test strip for testing an analyte in a liquid sample. The test strip includes a first support sheet, where a sample pad, a label pad and a test pad are sequentially arranged on a front face of the first support sheet from upstream to downstream; the label pad includes a label that is capable of flowing with a liquid; the test pad includes a fixed binding substance that is incapable of flowing with the liquid; and a layer of hydrophobic medium is coated on a back face of the first support sheet, to prevent the liquid sample from flowing from the back face of the first support sheet and wetting the test pad or the label pad in a downstream area in advance when the sample pad contacts the liquid sample.

## Description

### CROSS-REFERENCE TO RELATED APPLICATIONS

This application claims priority to Chinese Patent Application, Application No.: 202411328176.X, filed on September 23, 2024;202411323820.4, filed on September 23,2024 and all disclosures of this application are incorporated by reference in their entirety as a part of this application.

### BACKGROUND OF THE INVENTION

### Field of the Invention

The invention relates to the technical field of immunochromatography, and in particular, to a lateral flow test strip including a label pad.

### Description of the Related Art

The following background is used to help readers understand the invention and should not be considered as prior art.

It becomes a very common method to collect a liquid sample, such as urine, by using a testing device, and judge the presence of a specific analyte (such as drugs and/or metabolites thereof, or labels associated with diseases). Such testing device generally requires that the sample should be collected in a sample container, and a relevant technician should insert a test strip, submerge part of the test strip into the sample, then take out the test strip and read the result. The technician may come into contact with the sample, which may endanger his/her health or contaminate the sample. To avoid such risk, it is necessary to add a closed lid to the sample collection container before the operation.

At present, there are a plurality of closed devices, for example, the devices disclosed in US patents US 4,976,923, US 5,429,804, and US 6,726,879. The above devices fix the testing strip to the lid of the testing device. In use, the container is turned over or tilted to make the sample infiltrate the test strip and get ready for the test. The US patent application US2003/0027359A1, published on February 6, 2003, discloses a urine test cup. After the opening of the urine test cup is covered by the cover, a post piston still needs to be pushed to move by a pushing rod, such that the liquid sample flows out of a cup chamber and wets the testing element. Patent application 200510113977.5 published in China discloses a urine test cup. After the opening of the urine test cup is covered by the cover, the liquid flows from a collecting chamber to a testing chamber, and then test is started. Another patent application 200480033286.8 published in China also discloses a urine test cup. After the opening of the urine test cup is covered by the cover, test is started.

As many other sample collecting and testing devices are inefficient in extracting samples from the collection devices, they always have many problems, such as environmental pollution due to sample leakage, or testing results affected due to too few or too many samples collected, or complicated detection due to many operation steps. Many of these devices are also very complicated in their design and manufacture, for which quite expensive materials need to be used. Therefore, better methods and devices are needed to collect and detect samples.

Recently, such testing devices have been increasingly used by ordinary families or non-professional institutions. As these testing device are specially designed for non-professionals, they need to simplify operation processes and ensure the accuracy of testing results. Therefore, testing devices with simple operation and accurate and reliable testing results are a necessity of the society at present.

At present, test strips or test cards and other testing materials developed with colloidal gold immunochromatography are widely used in medicines or ordinary people's homes. With these simple and fast test strips or test cards, for example, colloidal gold test strips for early pregnancy detection, ordinary people can obtain the testing results within a minute or ten-odd minutes at most. Therefore, it is of great significance to further improve these test strips or test cards.

A transverse test strip is in contact with the liquid sample alone or by setting on a carrier, which absorbs liquid under capillary actions and allows the liquid to flow over the test strip to complete the test. Such test strip often includes an absorbent pad for contact with the liquid, a label pad to receive the liquid from the absorbent pad and dissolve the label on the absorbent pad, and a test pad that flows downstream to complete the test. The most important thing about the test strip is that the label on the label pad is dissolved due to the humidity of the test pad. During liquid test, the most critical problem is the flood phenomenon. The liquid is expected to flow sequentially in strict order. However, a variety of factors cause some liquid to flow directly to the test pad without passing through the label pad, while moistening the test pad in advance results in failure to complete the test or inaccurate test. This phenomenon is even more prominent in the home test.

Therefore, it is necessary to improve the existing test strip, so as to meet different test needs, make the testing results more accurate, and make the test strip especially suitable for home test, with simpler operation and no influence on the test structure.

### BRIEF SUMMARY OF THE INVENTION

The invention aims to provide a lateral flow test strip with higher sensitivity. The use of the test strip can overcome a problem of inaccurate testing results caused by excessive liquid flowing onto the test strip during the test of large-volume urine.

According to a first aspect, the test strip disclosed in the invention includes a sample receiving pad, a label pad, a test pad and a first non-absorbent support sheet that are sequentially arranged from upstream to downstream, including an upper surface and a lower surface, where the sample pad, the label pad and the test pad are all arranged on the upper surface of the first support sheet, and the lower surface of the first support sheet is coated with a hydrophobic medium. The hydrophobic medium includes a hydrophobic adhesive. When such a test strip is arranged in a clamping slot on a carrier, the liquid will not flow quickly through a capillary gap formed between the back face of the first support sheet and the bottom surface of the clamping slot to wet the test pad in advance. Because the hydrophobic medium is an adhesive layer, the adhesive layer is bonded to the bottom of the clamping slot; the capillary gap is absent between the adhesive layer and the bottom of the clamping slot, and naturally, no liquid flows between them. Therefore, the liquid is allowed to flow from the sample pad and the label pad as much as possible.

In some embodiments, the label pad includes a label area and the sample receiving pad, and the sample receiving pad and the label area are made of a same material. In some embodiments, a second non-absorbent support sheet is provided below the first support sheet, and arranged below a superimposing position of the label pad and the test pad. In some embodiments, a part of the second support sheet is arranged below the test pad and the other part of the second support is arranged below the label pad. In some embodiments, the second support sheet includes two ends, a first end and a second end, where the first end is arranged below the sample receiving pad and the second end is arranged below the test pad. In some embodiments, a distance between the first end of the second support sheet and the label area of the label pad is smaller than a distance between the first end of the test pad and the label area. Alternatively, a length of the second support sheet is less than a sum of lengths of the label pad and the sample receiving pad. The second support sheet being arranged below the first support sheet is equivalent to locally increasing the thickness of the test strip, so the second support sheet is allowed to contact with the bottom of the clamping slot. Even if the liquid flows from a gap between the second support sheet and the bottom surface, because a distance between the sample pad, the label pad or the test pad and the bottom surface increases, the liquid can flow in the gap and then flow to these label pads or test pads to in advance wet them.

In some embodiments, the second non-absorbent support sheet includes one or more scribed lines to divide the second support sheet into one or more bendable areas. In some embodiments, the second support sheet is bonded to the lower surface of the first support sheet by a hydrophobic medium. In some embodiments, the second support sheet is bonded to the lower surface of the first support sheet by the hydrophobic adhesive. In some embodiments, the lower surface of the second support sheet is also coated with a layer of hydrophobic medium or hydrophobic adhesive. In some embodiments, the hydrophobic adhesive layer is also covered with a non-absorbent membrane.

In some embodiments, the test strip further provides a first non-absorbent covering layer that is a flexible non-absorbent membrane; and the covering layer covers a part of the sample absorption pad, and completely covers the label pad and partially covers the test pad. In some embodiments, one end of the flexible covering lay covers the test pad and is proximal to the label area. In some embodiments, the test pad further includes a second covering layer (rolling and stretching layer), and the second covering layer covers the first covering layer. In some embodiments, the second covering layer is a rigid non-absorbent layer. In some embodiments, the rigidity of the second covering layer is greater than that of the sample receiving pad, the label pad and the test pad. In some embodiments, the first covering layer and the second support sheet are made of a same material. In some embodiments, the second non-absorbent covering layer includes one or more scribed lines to divide the second support sheet into one or more bendable areas. Therefore, when a plurality of label pads are provided, they necessarily have a specified thickness or height, and the sample pad and the plurality of label pads are superimposed together and have a specified gradient or distance difference, such that a covering element can be bent or folded to fit with the structure of the test strip and plays a role in tightly combining the label pad and the sample receiving pad and tightly combining the label pad and the test pad. The positions of the label pad and the test pad are kept unchanged, and the capillary force on the liquid is kept unchanged.

Further, the first covering layer includes a self-adhesive section; the self-adhesive section is used for bonding the first covering layer together with other components to play a fixing role; and the sample pad can be made of a non-woven fabric, a glass fiber and other materials, and is the sample addition part.

Preferably, the first covering layer is provided with the second covering layer (rolling and stretching layer), such that label pads can be stacked more tightly, and the number of the strips is greater than or equal to 2 and the strips may be PVC strips or PS strips.

In some embodiments, the label pad includes a colored part and a white part. The colored part is a place with a label, and the white part does not have the label. The label pad is generally made of colloidal gold, latex, and the like coated on the non-woven fabric or the glass fiber. The colloidal gold is an aqueous solution of chloroauric acid, has high electron density and can be combined with a variety of biological macromolecules. It is a non-radioactive tracer commonly used in immunolabeling technologies. Preferably, a length of the white part is 2-8 mm. In some embodiments, a plurality of label pads are provided and superimposed on each other. Preferably, the label pads are stacked together in a straight line, and the white part of one of the label pads is superimposed on an upper side of the colored part of another adjacent label pad. Further, a superimposing length of the white part of one of the label pads and the colored part of another of the label pads is no more than 6 mm. Further, after the white part of one of the label pads is superimposed with the colored part of another of the label pads, its colored part can be further superimposed with the colored part of another of the label pads, and a superimposing length of the colored part and the colored part of another of the label pads is no more than 6 mm. Further, a superimposing length of the colored part of one of the label pads and the colored part of another of the label pads is no more than 5 mm.

Preferably, the second covering layer can cover all the label pads, so as to protect the label pads and allow them to be more tightly bonded. In some embodiments, the label pads have a same label thereon. In some embodiments, the label in the label area includes an antibody that specifically binds to the analyte in the sample and colored particles conjugated with the antibody. In some embodiments, the label pad includes a colored part sprayed with the label and a white area without the label. In some embodiments, a length of the white area is 2-8 mm. In some embodiments, a length of the colored part is 2-8 mm. In some embodiments, the plurality of label pads are stacked together end to end in a straight line, and the white area of one label pad of the plurality of label pads is partially superimposed on the colored part of another adjacent label pad. Further, a superimposing length of the white part of one of the label pads and the colored part of another of the label pads is no more than 6 mm. Further, a superimposing length of the white part of one of the label pads and the colored part of another of the label pads is no more than 5 mm. Further, a superimposing length of the white part of one of the label pads and the colored part of another of the label pads is no more than 4 mm.

In some embodiments, the label pad includes 3-8 label pads. In some embodiments, the label pads are sequentially arranged from upstream to downstream. In some embodiments, the label includes gold particles or latex particles. In some embodiments, the antibody on the label pad is a specific binding THC antibody, and THC antigen substances are provided on the testing area on an NC membrane. In some embodiments, the label pad includes 4 label pads superimposed end to end.

In some embodiments, the antibody on the label pad is a first antibody specifically binding to HCG, and a second antibody specifically binding to HCG is provided on the testing area on the NC membrane. In some embodiments, the label pad includes 5 label pads superimposed end to end. In some embodiments, the label pad includes 4 label pads superimposed end to end. In some embodiments, one end of the second covering layer covers the sample pad, and the other end thereof is connected with the NC membrane. In some embodiments, the sample pad is made of a non-woven fabric or a glass fiber.

According to a second aspect, the invention provides a testing device, namely a device for testing an analyte in a sample. The device includes a carrier, where the carrier includes a front face and a back face; one or more clamping slots for accommodating a test strip are provided in the front face, the test strip is used for testing the analyte in the liquid sample and includes a sample pad, a label pad and a test pad that are sequentially arranged from upstream to downstream, and the clamping slot is provided with a bottom and an opening; and a first covering layer, where the first covering layer includes a first covering area and a first blocking area, the first covering area covers the one or more clamping slots to form one or more channels with one end being sealed and the other end being open, a part of the sample pad is exposed from the opening of the channel, and the first blocking area covers the surface of the partially exposed sample pad.

In some embodiments, the first blocking area of the first covering layer includes the front face and the back face, the back face includes an adhesive layer, and the first blocking area is bonded to a surface of the part of the sample pad through the adhesive layer. In some embodiments, the sample pad extends outward from the first blocking area instead of being covered by the first blocking area, and the sample pad not covered by the first blocking area is arranged to contact the liquid sample. The provision of such a blocking area is to block or delay the liquid sample from entering the channel through the opening of the channel, so as to prevent the liquid from flowing to the test pad or the label pad through the capillary gap formed between the back face of the test strip and the channel in advance. In other words, the velocity of the liquid flowing through the gap is greater than the velocity of the liquid flowing on the sample pad and the label pad, thus wetting the label pad or the test pad in advance, resulting in the failure or inaccuracy of the test.

In some embodiments, the carrier further includes a second covering layer covering the back face of the carrier, and the second covering layer includes a second blocking area, and the second blocking area covers a back face of the part of the sample pad. The partially exposed sample pad is covered by the first covering layer and the second covering layer. It can be understood that the membrane covering the clamping slot extends longitudinally to cover a part of the exposed sample pad.

In some embodiments, the second blocking area further includes the adhesive layer, and the second blocking area is bonded to the back face of the sample pad through the adhesive layer. In some embodiments, when the carrier includes a plurality of clamping slots, each of some of the clamping slots includes a test strip, a spacing area is arranged between each test strip, and the first blocking area and the second blocking area are bonded to each other in the spacing area. Generally, the sample pad of the test strip extends outward from the opening of the channel, and a closed area is formed at the front end of the opening through the blocking area; a liquid is not easy to flow into the closed area, and of course, difficult to enter the opening, so no liquid will flow through the capillary gap formed by the back face of the test strip and the bottom of the clamping slot.

In some embodiments, a width of the first blocking area and a width of the second blocking area are greater than a sum of widths of all exposed sample pads. The sample pads of each test strip here are spaced from each other or have spacing areas, so the sum of the widths of all the exposed sample pads includes the width of the spacing areas. Therefore, the two blocking areas have an area exceeding the sum of the widths in a lateral direction, and the exceeded areas can also be bonded to each other. This prevents liquid from entering the opening of the channel from the side of the test strip. Thus, in some embodiments, the first blocking area and the second blocking area are bonded to each other at a side edge of the sample pad, thereby preventing the liquid sample from flowing into the opening from the side edge of the sample pad.

In some embodiments, the first blocking area and the second blocking area are flexible membranes. In some embodiments, the first covering layer and the second covering layer are made of a same material, and the first covering area and the first blocking area are made of the same material, so they cannot be zoned only according to the function. In some embodiments, the flexible membranes are non-absorbent membranes. It can be understood that a membrane with a thickness of about 1 mm or 0.5 mm covers the part of the sample pad, and is bonded to each other from the back face and the front face. This is equivalent to a fact that all sides of the test pad are wrapped. The function of such wrapping is to prevent the liquid from directly flowing into the opening of the channel, but only to make the liquid flow by the capillary action of the test strip itself.

In some embodiments, the device further includes a chamber for accommodating the carrier, and the chamber includes an opening for receiving a liquid sample. In some embodiments, the chamber includes a bottom, side walls and an opening, the carrier leans against the side walls, and an end of a part of the sample pad is proximal to the bottom of the chamber, such that after the liquid sample enters the chamber, the sample pad is capable of contacting the liquid sample at the bottom of the chamber.

In some embodiments, if some treatments are made to the back face of the test pad, the liquid can be better prevented from flowing in the capillary gap between the back face and the bottom surface of the clamping slot. For example, a layer of hydrophobic medium such as a hydrophobic adhesive is coated. In some embodiments, a second support sheet is bonded onto the hydrophobic adhesive. The sample pad or the label pad of the test strip is allowed to be raised through the second support sheet. Therefore, even if the liquid enters the opening and flows through the gap between the back face of the second support sheet and the bottom of the clamping slot, the liquid is not easy to flow into the label pad because the lab pad is raised by the second support sheet. If the test pad is raised by the second support sheet, the liquid will not flow onto the test pad. Thus, in some embodiments, the second support sheet is located below the sample pad and/or the label pad. In some embodiments, the second support sheet is located below the sample pad and the label pad. In some embodiments, the second support sheet includes ends, a first end is located below the label pad, and a second end is located below a superimposing position of the label pad and the test pad. In some embodiments, the first end of the second support sheet is shorter than an end of the sample pad. In some embodiments, a back face of the second support sheet also includes a layer of hydrophobic adhesive.

### BRIEF DESCRIPTION OF THE DRAWINGS

FIG. 1 is a schematic diagram showing a structure of a test strip according to a specific embodiment of the invention.
FIG. 2 is an exploded view of a test strip according to a specific embodiment of the invention.
FIG. 3A is a standard colourimetric card for determining a color depth of T line on a test pad.
FIG. 3B shows a test strip according to a specific embodiment of the invention; when a capillary gap is formed between a back face of a first support sheet 111 and a surface 301 of a bottom plate, a liquid can flow rapidly through the capillary gap to wet a part of an area 201 on a test pad in advance.
FIG. 4 is an exploded diagram showing a structure of a test strip according to a specific embodiment of the invention.
FIG. 5 is an exploded diagram showing a structure of a test strip according to a specific embodiment of the invention.
FIG. 6 is an exploded diagram showing a structure of a test strip according to a specific embodiment of the invention.
FIG. 7 is an exploded diagram showing a structure of a test strip according to a specific embodiment of the invention.
FIG. 8A is a schematic diagram for principle interpretation of an embodiment of a conventional technology according to the invention (a liquid in advance wets different flow paths with the change of flow velocity).
FIG. 8B is a schematic diagram for principle interpretation of an embodiment of a conventional technology according to the invention (uneven materials of a sample pad and a label pad lead to different area differences in a flow velocity of a liquid).
FIG. 8C is a schematic diagram for principle interpretation of an embodiment of a conventional technology according to the invention (uneven materials of a sample pad and a label pad lead to different area differences in a flow velocity of a liquid).
FIG. 9A is an exploded diagram showing a structure of a test strip according to a specific embodiment of the invention.
FIG. 9B is a partially enlarged diagram of an assembled test strip shown in FIG. 9A according to the invention.
FIG. 10A is a schematic diagram showing an exploded structure of a carrier in a testing device according to a specific embodiment of the invention.
FIG. 10B is an enlarged schematic diagram of a partial structure of a carrier according to a specific embodiment of the invention.
FIG. 10C is a schematic diagram of a combined three-dimensional structure of a carrier including a test strip according to a specific embodiment of the invention.
FIG. 10D is a partially enlarged schematic diagram of a combined carrier according to a specific embodiment of the invention.
FIG. 10E is a structural schematic diagram of a carrier including a front face of a test strip.

### DETAILED DESCRIPTION OF THE INVENTION

The structures involved in the invention or the technical terms used are further explained below. Unless otherwise specified, they shall be understood and explained according to the general terms commonly used in the art.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein, or a polymer. In addition, detection means that the amount of a substance or material is tested. Further, assay also means immunoassay, chemical assay, enzyme assay, and the like.

### Samples

The samples detected or collected by the testing device of the invention include biological liquid (for example, case liquid or clinical sample). Liquid samples or fluid samples may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other wastewater.

An appropriate testing element according to the invention can be used to detect any analyte. Preferably, the testing device of the invention is used to detect small drug molecules in saliva and urine. Preferably, the testing device can be used to detect small molecular substances such as viruses and bacteria in saliva, throat or nasal fluid. Any samples of the above forms may be collected using a collector (not shown) according to the invention, regardless of being solid or liquid at the beginning, provided that these liquids or liquid samples can be absorbed by an absorption element generally located on the collector. Generally, the absorption elements here are made of absorbent materials, dry at the beginning, and can absorb liquid samples or fluid samples through the capillary or other characteristics of the absorption element materials, such that the fluid samples can be kept in the absorption elements. The absorption material may be any material capable of absorbing liquid, such as sponge, filter paper, polyester fiber, gel, non-woven fabric, cotton, polyester membrane, yarn, and flocking. When flocking swabs are used, flocking swabs as described in the following patents can be used as a part of the present invention: US 8,114,027, US 8,317,728, US 8,979,784, US 9,011,358, US 9,173,779, US 10,327,741, AU 2004226798, JP 4579902, and ZL 200610099310.9. In some embodiments, the dry absorption element is hard; for example, the wet sponge becomes soft, and then can be compressed, thus releasing liquid. Of course, a sparse sponge, such as a sponge swab, can also absorb a small amount of liquid samples, such as 5-100 µL. For example, a sponge cotton swab described in U.S. Provisional Application 63/300,811, filed on Jan. 19, 2022, can also be used in the invention as a specific embodiment of the collector.

Of course, the absorption element may be made of an absorbent material or a non-absorbent material. However, the absorption element is provided with holes, screw threads, and caves on which the samples can be collected. Generally, the samples are solid or semi-solid samples, and filled between screw threads and in the holes or caves for collection. Of course, optionally, the absorption element may be composed of some non-absorbent fibers and hairs, and these materials are used to scrape a solid, semi-solid or liquid sample, such that these samples can be retained on the absorption element.

### Downstream and upstream

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, the testing element mentioned in the invention has a sample pad 104, a label pad 103, a test label 102 and an absorption pad 101. The sample pad is located upstream of the label pad, the test pad is located downstream of the label pad, and the absorption pad is located downstream of the test pad. Generally, the liquid flows along the testing element from upstream to downstream.

### Gas communication or liquid communication

Gas communication or liquid communication means that liquid or gas can flow from one place to another. In the flow process, the liquid or gas may pass through some physical structures that play a guiding role. The "passing through some physical structures" here means that liquid passes through the surface of these physical structures or their inner space and flows to another place passively or actively, where passivity is usually caused by outer forces, such as flow under the capillary action and the action of air pressure. The flow here may also be a flow due to self-action (gravity or pressure) of the liquid or gas, and may also be a passive flow. The fluid under the action of air pressure may be a forward flow, or also a reverse flow; or a fluid is caused to flow to another position from a position under the action of air pressure. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. Alternatively, two objects are separated at the beginning, combined to achieve a specified function if necessary, then separated, or later combined again for a purpose. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

### Analyte

In examples where the analyte according to the invention can be used, some semiantigen substances are involved and include drugs (such as drugs of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of abuse of drug include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used to detect drugs belonging to a medical use but easy to be taken excessively, such as tricyclic antidepressants (imipramine or analogues) and acetaminophen. These drugs are metabolized into different micromolecular substances after being absorbed by human body. These micromolecular substances exist in blood, urine, saliva, sweat and other body fluids or in some body fluids.

For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Campylobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the invention.

### Testing element

The "testing element" used herein refers to an element that can be used to detect whether a sample or a specimen contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the invention.

Various testing elements can be combined for use in the invention. One form of the testing elements is a test strip or a lateral flow test strip. The test strips used for analyzing the analyte (such as drugs or metabolites that show physical conditions) in samples can be of various forms such as immunoassay or chemical analysis. A non-competitive or competitive analysis mode may be used for the test strips. A test strip generally contains an absorbent material that has a sample application area, a reagent area, and a testing area. Fluid or liquid samples are added to the sample application area and flow to the reagent area under the capillary action. If analyte exists in the reagent area, samples will bind to the reagent. Then, the samples continue to flow to the testing area. Other reagents such as molecules that specifically bind to the analyte are immobilized in the testing area. These reagents react with the analyte (if any) in the sample and bind to the analyte in this area, or bind to a reagent in the reagent area. The label used for displaying the detection signal exists in the reagent area or the detached label area.

In a typical non-competitive analysis mode, if a sample contains the analyte, a signal will be generated; and if not, no signal will be generated. In a competitive method, if no analyte exists in the sample, a signal will be generated; and if the analyte exists, no signal will be generated.

The testing element may be a test strip, which may be made of an absorbent material or non- absorbent material. The test strip may contain several materials used for delivery of liquid samples. One material of the test strip can cover the other material thereof. For example, the filter paper covers the nitrocellulose membrane. One or more materials may be used in one area of the test strip, and one or more other different materials may be used in the other area. The test strip can be stuck to a certain support or on a hard surface for improving the strength of holding the test strip.

The analyte is detected through a signal generating system. For example, one or more enzymes that specifically react with this analyte is or are used, and the above method of fixing a specific binding substance on the test strip is used for fixing the combination of one or more signal generating systems in the analyte testing area of the test strip. The substance that generates a signal may be in the sample application area, the reagent area or the testing area, or on the whole test strip, and one or more materials of the test strip may be filled with this substance. The solution containing a signifier is added onto the surface of the test strip, or one or more materials of the test strip is or are immersed in a signifier-containing solution. The test strip containing the signifier solution is dried.

Various areas of the test strip may be arranged in the following way: a sample application area, a reagent area, a testing area, a control area, an area to determine whether the sample is adulterated or not, and a liquid sample absorption area. The control area is located behind the testing area. All areas can be arranged on one test strip that is only made of one material. Alternatively, different areas may be made of different materials. Each area can directly contact with the liquid sample, or different areas are arranged according to the flow direction of liquid sample; and a tail end of each area is connected and superimposed with the front end of another area. Materials used can be those with good water absorption such as filter paper, glass fibers or nitrocellulose membranes. The test strip may also be in other forms.

The nitrocellulose membrane test strip is commonly used, that is, the testing area includes a nitrocellulose membrane (NC) on which a specific binding molecule is immobilized to display the testing result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or testing device of the invention for the test of an analyte, for example, the test of an analyte in a sample.

Test strips used in the invention may be commonly referred as lateral flow test strips. The specific structure and detection principle of the test strips are well known to a person skilled in the art in the prior art. A common test strip includes a sample collection area or a sample application area, a label area, a testing area and a water absorption area. The sample collection area includes a sample receiving pad, the label area includes a label pad, and the water absorption area may include an absorbent pad. The testing area includes necessary chemical substances for detecting the presence or absence of the analyte, such as immunoreagents or enzyme chemical reagents. The nitrocellulose membrane test strip is commonly used, that is, the testing area includes a nitrocellulose membrane, and a specific binding molecule is immobilized on the nitrocellulose membrane to display the testing result; and other test strips such as cellulose acetate membrane or nylon membrane test strips may also be used. Of course, in the downstream of the testing area, there may also be a testing result control area. Generally, the control area and the testing area are in the form of horizontal lines, namely, a test line or a control line. Such test strips are conventional. Of course, they can also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the testing device of the invention or can be disposed in contact with the liquid samples in a testing chamber or used for detecting the presence or absence of an analyte in the liquid samples that enter a testing chamber, or the quantity thereof.

In addition to the foregoing test strip or lateral flow test strip which is used for contacting with the liquid sample to test whether the liquid samples contain analytes. The testing element of the invention may be used as a testing device by itself to detect an analyte in a sample. Therefore, the testing device here is equal to a testing element. For example, after mixed with a treatment solution, the fluid sample is detected with the testing element directly, specifically described as follows: when a receiving device is described to treat a fluid sample, the testing element may be used for detection alone.

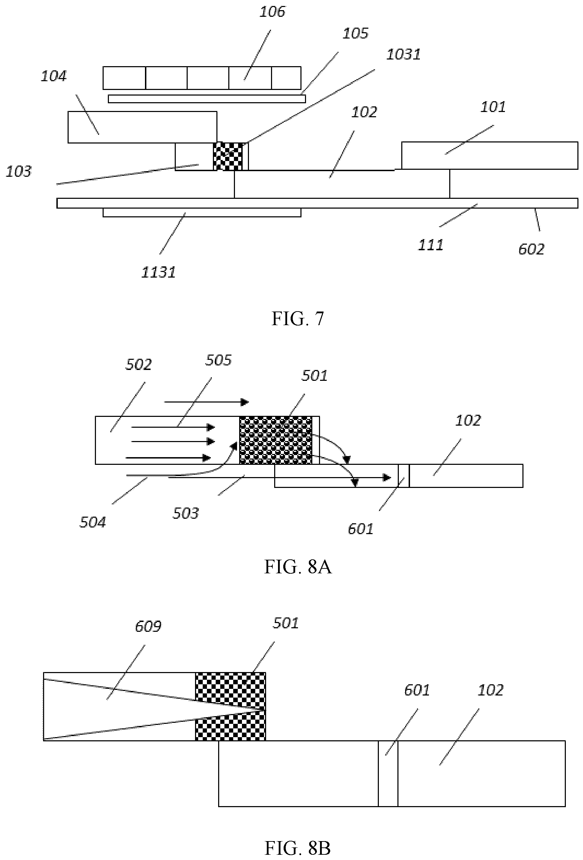

In some embodiments, the invention provides an improved test strip. As shown in FIG. 7, the test strip includes a first support sheet that is a non-absorbent support sheet; a liquid sample receiving pad (which can also be named as "sample pad") 104, a label pad 103, a test pad 102, and an absorbent pad 101 are sequentially arranged on the front face of the support sheet from upstream to downstream, and these pads are superimposed on each other. For example, one end of the liquid sample receiving pad 104 is superimposed on one end of the label pad, and the other end of the liquid sample receiving pad 104 is used for receiving a sample, such as a liquid sample. The end of the label pad 103 with the label area 1031 is superimposed on the test pad, a test line and a testing result control line are provided on the test pad, the immobile antibody or antigen is immobilized on the test pad, and a label compound with colored particles is treated on the label area and includes the antibody or antigen and can move with the liquid. Generally, the test line is located downstream of the label area 1031 of the label pad 103. When the liquid contacts the sample pad 104, the liquid flows through the sample pad 104 and then flows to the downstream label pad 103. The liquid dissolves the dry label 1031 in the label area on the label pad, and then drives the label to flow to the downstream testing area to react with the immobile antibody or antigen on the testing area. Colored lines or colorless lines are formed in the area on the test pad, indicating the testing results. When a competition method is adopted, the testing results are positive in case of the colorless lines, and the testing results are negative in case of the color lines. When a sandwich method is adopted, the testing results are negative in case of the colorless lines, and the testing results are positive in case of the color lines. The label here is generally colloidal gold particles conjugated with antibodies or antigens, and is in a form of a solution, and is coated on the label pad or sprayed on the label pad by a machine, and then dried.

When the liquid sample contacts the sample pad 104 and flows to the test line of the testing area through the label pad, the liquid reacts in a flowing process and then results are tested. In addition, the capillary force of each pad is used to induce or pull the flow of the liquid. Therefore, the flow velocity of liquid is determined by the capillary force. The liquid dissolves dry substances on the sample pad 104 and the label pad 103 during flowing, for example, substances for improving testing properties and labels for displaying color, and then flow to the test pad to react with immobilized antibodies and antigens. However, the capillary force only exists in dry porous materials. If the dry porous materials get wet, they will lose the capillary force and will not provide a driving force for the liquid; alternatively, if some areas get wet in advance, the flow velocity of liquid will slow down and no enough driving force is provided to make the liquid flow.

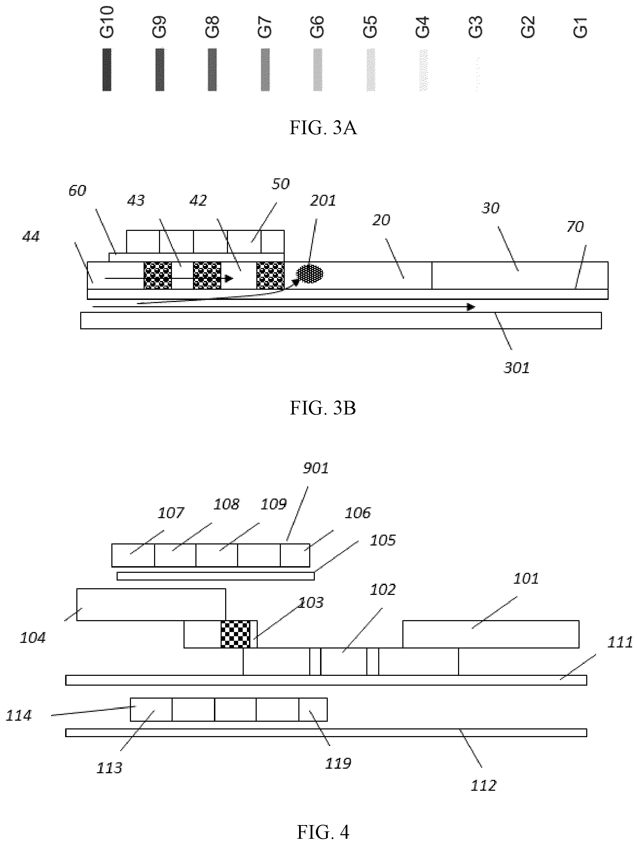

However, in a specific operation, except that some liquid flows by the capillary force on the test strip, some liquid does not flow to the test strip completely by the capillary force, which may wet some areas in advance, for example, the label pad. Therefore, this will result in failure of later liquid to wet such areas, and the later liquid may carry necessary substances for detection. However, when it passes through those wet areas, binding reaction cannot occur, resulting in the error of the testing results. This is more complicated in an operating environment of home self-testing. After all, a home self-tester has no professional operating experience with randomness of self-testing, this leads to the inaccuracy of the testing results. In a conventional test strip as shown in FIG. 3B, when the back face of the support sheet 70 directly contacts the surface 301 of the bottom plate, the liquid can be allowed to flow through the capillary gap between them and can flow to the testing area 201 in advance and wet it. The test pad has the first covering layer 60 and the rolling and stretching layer 50, so the liquid from the label pad cannot flow to the test pad to complete the test. Even if the liquid flows on the test strip by the capillary force, the sample receiving pad and the label pad between the test strips cannot be guaranteed to have 100% consistent materials. For example, in a same batch of test strips, although the sample pads are made of same materials, their properties are different, for example, pore size distribution, pore size and thickness cannot be guaranteed to have 100% consistency. Similarly, although the label pads are made of same materials, 100% consistency between each label pad on each test strip cannot be guaranteed, for example, thickness and pore size distribution directly affect the flow properties of liquid, such as the flow velocity of liquid and circulation paths for liquid diffusion. Especially, for some special analytes, such as THC, the adsorption property of the analytes is reduced, and sample pads and label pads are made of non-adsorptive materials, and the materials are loose, large in pore size and uneven in distribution. Since there are differences between these sample receiving pads and label pads of each test strip, how to ensure that the differences will not substantially affect the testing results and how to eliminate the differences by some ways are an insurmountable problem for conventional test strips, and such a material difference will also affect the flow velocity of liquid. Therefore, when the material difference and the liquid flow under the non-capillary force coexist, the testing inaccuracy will be more serious. For example, as shown in FIG. 8A, the test strip includes a label pad 502 with a label area 501 on which a dry label is provided, the label pad is superimposed on the test pad 102, the test pad is generally an NC membrane (nitrocellulose membrane), and a test line 601 of an antibody or antigen is immobilized on the membrane. During the test, on the one hand, a liquid flow path 503 is that the liquid flows from the sample pad to the test pad 102 along the test direction without passing through the test strip, and the flow velocity of liquid is faster than that of the liquid on the test strip, such that the liquid flow path 503 may reach the test pad 102 in advance, or reach the label area 501 in advance to dissolve the label, and then flow onto the test pad 102. In this case, the liquid arriving in advance wets the test pad 102 in advance, and when the liquid from the label pad passes through the wet area again, the reaction cannot be completed, and after all, the area gets wet in advance. In addition, the liquid sample flowing from the liquid flow path 504 does not flow through the test strip at the beginning, but enters the label pad 502 halfway, dissolves a mixed solution formed by the label (No. 1 mixed solution) and finally enters the testing area. The liquid sample flowing along the liquid flow path 505 completely passes through the sample pad, and then flows to the label pad. In the label area, No. 2 mixed solution (normal flow) for dissolving the label and No. 1 mixed solution do not include same substances, thereby leading to a great difference in the testing results of the testing area. For the same liquid, some testing results are positive, while some testing results are negative. This will result in inaccurate testing results.

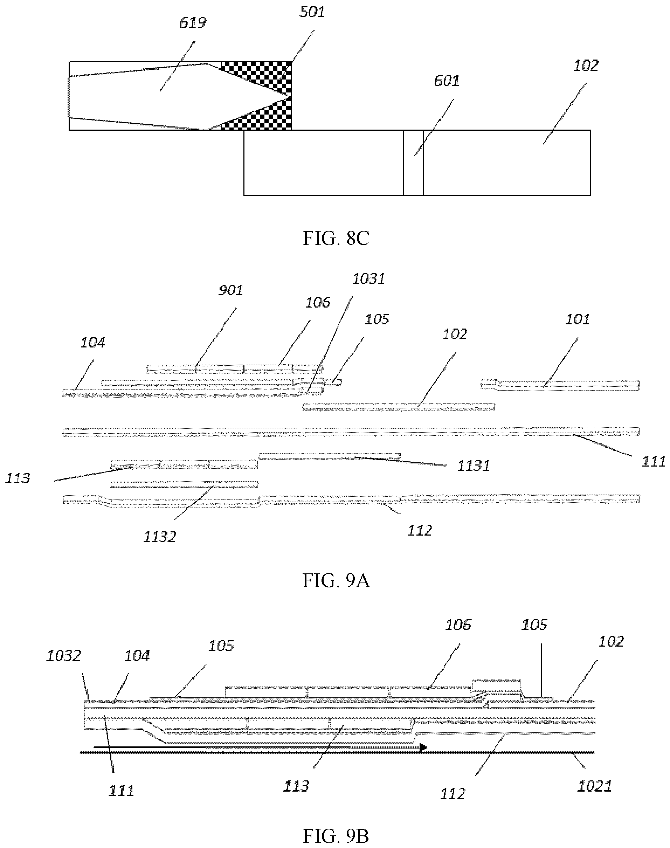

On the other hand, although all liquids flow from the test strips, they may flow fast and evenly on some test strips. However, on some test strips, some liquids flow fast and some liquids flow slowly. The liquid flow paths on different test strips are not uniform, but one same test strip has multiple liquid flow paths with different flow velocity. For example, as shown in FIG. 8B and FIG. 8C, a liquid flow path 609 is present in the label area on the test strip, a liquid flow path 619 is respectively present on different test strips or sometimes in the label area 501 on the test strip. When there are two different liquid flow paths, the paths and areas through which the liquid passes are different, resulting in inconsistent liquid flow. These reasons are caused by the different properties of the sample pad and the label pad between the test strips even if they are made of same materials.

In order to solve the above problems and make the testing results more accurate, especially in home test, the invention provides a simple method to solve them. For example, as shown in FIG. 7, a layer of hydrophobic medium 1131 is directly coated on the back face of the non-absorbent support sheet (the first support sheet 111), and can prevent the liquid from flowing downstream along the test strip from the back face 602 of the support sheet 111, thus reducing the liquid from flowing downstream along the back face of the test strip. Especially, such a test strip is placed in the clamping slot of the carrier, and generally, the back face 602 of the support sheet 111 is proximal to the bottom surface of the clamping slot in the carrier, and there is a gap between them. However, if the hydrophobic medium is coated on the first support sheet 111, at least the liquid cannot flow directly downstream from the back face, which reduces the chance that the liquid directly wets the area on the test strip in advance before the liquid reaches it. When the flow velocity of liquid on the back face of the support sheet 111 is faster than that on the test strip, the liquid may flow to the label pad or the test pad in advance and wet these areas in advance, such that the areas lose the capillary force and then slow down the flow of the later liquid or prevent the later liquid from flowing, causing the failure of the test.

This is due to a fact that the liquid sample flows directly from the back face of the test strip to the downstream, and the flow velocity is much faster than that of the liquid sequentially flowing to the sample pad 104 on the test strip, the label pad 103 and the test pad 102. Thus, these liquids can wet the testing area 102, the label pad 103 or the absorbent pad 101 in advance, such that the absorbent pad loses its water absorption capacity, and the liquid from the label pad or the liquid flowing on the test pad 102 stops flowing or has slow flow velocity due to absence of enough capillary driving force. Thus, this will lead to the error of the testing results.

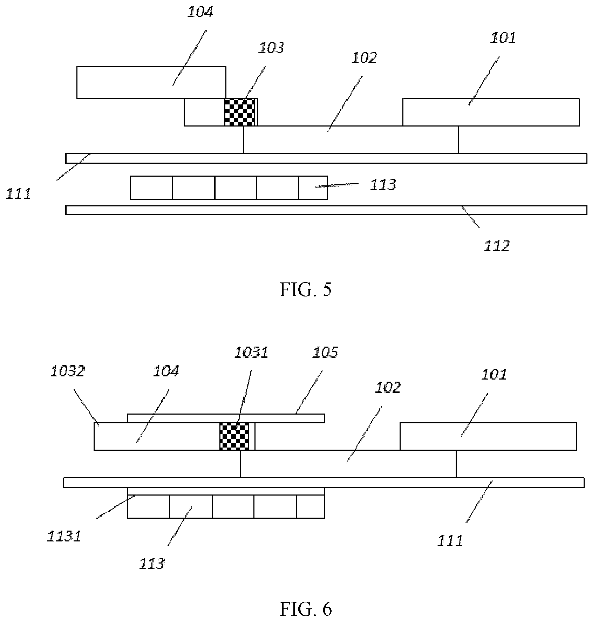

In addition, the process of coating a hydrophobic medium is troublesome. A layer of adhesive tape is directly bonded onto the back face 602 of the support sheet 111 and is a sticky and hydrophobic adhesive layer, such as double-sided adhesive tape, and can be directly bonded onto the back face 602 of the first support sheet 111. As shown in FIG. 4 - FIG. 6, in some embodiments, a second support sheet 113 is bonded on the hydrophobic adhesive layer, is also non-absorbent and has a specified thickness. As shown in FIG. 6, the material of the second support sheet may be the same as or different from that of the first support sheet 111, but both the materials are non-absorbent materials. Their thickness can be the same or different. This is equivalent to a fact that the first support sheet 111 is bonded onto the sample receiving pad 104 and the back face of the area of the label pad 103 is thickened; the thickened back face is equivalent to a dam, thereby preventing the liquid from flowing downstream along the back face of the test strip or even on the back face and preventing at least the flowing liquid from flowing to the label pad or the test pad. Because the second support sheet 113 has a thickness and is non-absorbent, it can prevent the liquid from flowing from the back face of the test strip. In some embodiments, the second support sheet can be arranged below the sample application pad 104, the label pad 103 or the test pad. In some embodiments, the second support sheet 113 may have two ends, namely a first end 114 and a second end 119. The first end 114 of the second support sheet 113 is located below the sample receiving pad 104, and the second end 119 thereof is located below the superimposing position of the label pad and the test pad. Here, "below" means below according to the spatial position. The test strip has the first support sheet 111; and the test pad, the sample absorption pad and the label pad that are directly arranged on the surface of the first support sheet 111. The second support sheet 113 is bonded onto the back face of the first support sheet and is also located below the test pad, the sample absorption pad and the label pad, but the second support sheet does not directly contact with the test pad, the label pad and the sample receiving pad. Therefore, it can be understood that the second support sheet 113 is bonded onto the back face of the first support sheet 111, and the sample receiving pad 104, the label pad 103 and the test pad 102 are directly bonded onto the front face of the first support sheet corresponding to the back face. In some embodiments, the first end 114 of the second support sheet 113 is located below a part of the sample pad, or the first end 114 thereof is shorter than one end of the sample pad, or the length of the second support sheet 113 is shorter than the length after the sample pad and the label pad are superimposed. The use of the second support sheet is to minimize the flow of liquid from the back face of the test strip to downstream or even if the liquid flows, it may not flow to the sample pad or the test pad. The second support sheet 113 is arranged on the back face of the first support sheet 111 and has the blocking function. When a large amount of liquid pours into the test strip, the second support sheet 113 is similar to a waterproof dam to prevent the liquid from flowing. The most important thing is to prevent the liquid from reaching the testing area or the label area in advance, and to wet the testing area or a part of the label area in advance, which will naturally lead to inaccurate testing results.

As shown in FIG. 9B (1021 represents the bottom surface of the clamping slot), when these test strips are arranged on the carrier 800 with the clamping slot 803, the second support sheet 113 directly contacts the bottom surface of the clamping slot, whereas the bottom surface of the clamping slot is generally planar structure. In this case, it is equivalent to that the second support sheet 113 contacts the flat bottom surface of the clamping slot and indirectly contacts the sample receiving pad 104 (FIG. 9B shows that the sample pad 104 and the label pad are integrated, where the sample pad includes the label 1031), and the test pad 102 is bonded onto the first support sheet 111, such that the back face of the first support sheet corresponding to the test pad 102 directly contacts the flat bottom surface 890 of the clamping slot 803. Therefore, when the test is carried out, the sample pad and the label pad are raised by the second support sheet. Even if the liquid flows through the gap directly formed by the second support sheet and the bottom surface 1021 of the clamping slot, the liquid is not easy to flow to the label pad for advance wetting because the sample pad and the label pad are raised by the second support sheet. In addition, because the second support sheet is non-absorbent, it also prevents the liquid from entering the clamping slot 803 from the back face of the test strip, and especially it can prevent the liquid from flowing into the test pad for advance wetting. Generally, the thickness of the first support sheet 111 is 2 mm, and the thickness of the second supporting sheet is 2-3 mm, for example, it may also be 2 mm. Of course, a raising structure can be arranged on the clamping slot, such that the sample pad and the label pad can be raised and the second support sheet 113 can be omitted. Theoretically, the above arrangement is possible, but in practice, the above arrangement is very difficult. The width of a plastic clamping slot is 2-5 mm, and the depth thereof is 2-3 mm. It is very difficult to form a slope with a height of 1-3 mm in such a narrow space, and this requires a relatively precise injection mold and very high cost. In the invention, the second support sheet 113 is simply added to the first support sheet 111, directly or indirectly raising the height of the sample pad and the label pad (relative to the test pad), thus solving the phenomenon of liquid flooding at low cost.

In some embodiments, a flexible non-absorbent membrane 112 is bonded onto the back face of the second support sheet 113 and used to protect the second support sheet 113 from being damaged or falling off the first support sheet 111, and also has the protection function to prevent the liquid from flowing directly to the test pad. In some embodiments, the second support sheet 113 is bonded onto the first support sheet 111 by the adhesive layer, and the adhesive layer here can be bonded in the form of the double-sided adhesive, so the double-sided adhesive has the bonding function and serves as a hydrophobic medium, which has the waterproofing function. Compared with the second support sheet or the first support sheet, the first support sheet and the second support sheets are generally not flexible, at least they are more rigid than the flexible membrane 112 and are not easy to bend or fold. The flexible membrane 112 is also bonded onto the second support sheet 113 by the hydrophobic adhesive.

The hydrophobic adhesive is a non-absorbent and non-hydrophilic adhesive. The hydrophobic adhesive can be divided into different types according to different chemical compositions. It has different bonding effects on different materials and can achieve high-strength bonding. Generally, the hydrophobic adhesive includes a polyurethane adhesive that is a hydrophobic adhesive and is often used to bond wood, metal, glass and other materials. Fluororubber adhesive is a hydrophobic adhesive that is mainly used to bond various tough materials. It has properties such as resistance to high temperature and chemical corrosion and can be used in harsh environment. Siloxane adhesive is a hydrophobic adhesive. It is commonly used to bond metals, plastics and other materials, and can achieve high-quality and high-strength bonding. These adhesives or viscose reagents can be purchased from the market, and many commercial products can be purchased and used. The so-called "hydrophobic" means being not hydrophilic. Generally, liquid samples include water, and hydrophobic adhesives can prevent water from flowing on them or on their surfaces, so the water cannot flow downstream, and the flow velocity of basically non-flowing liquid is different from that of the liquid flowing on the sample pad under the capillary force, reducing the need to wet downstream areas in advance, such as the label pads and the test pad.

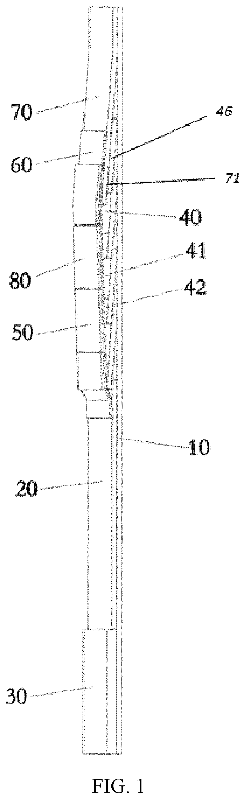

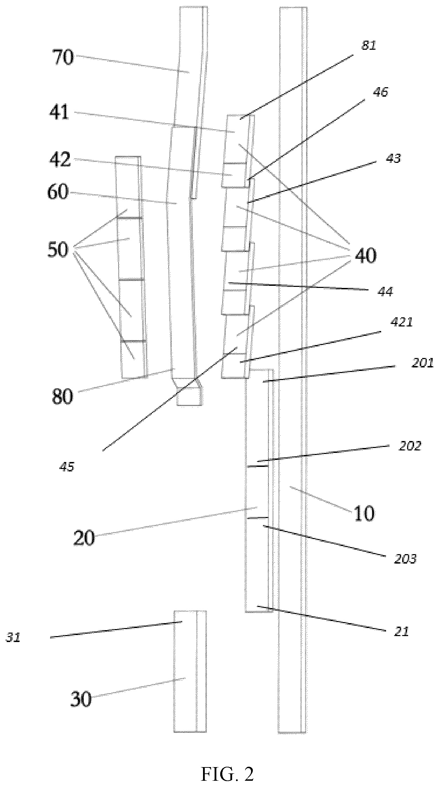

In some embodiments, the test strip provides a non-absorbent covering layer; and the covering layer covers a part of the sample application pad, and the label pad and covers the surface of a part of the test pad. As shown in FIG. 1 and FIG. 2, FIG. 3B, FIG. 4 and FIG. 6, after one end of the sample application pad 104 is superimposed on one end of the label pad 103, the other end of the label pad is superimposed on the test pad 102, and the superimposed part is located upstream of the test line 601. Thus, the first covering layer 105 (as shown in FIG. 6, FIG. 7 and FIG. 4), or the first covering layer 60 in FIG. 1 and FIG. 2 covers the downstream part of the superimposing position of the label pad and the test pad to the area of the part of the test pad, such that the label pad and the test pad can be fixed and more tightly combined (especially at the superimposing position), and the sample receiving pad and the label pad are in a steady unfolding state. The covering layer is an adhesive layer with an adhesive on a side thereof, and the first covering layer 105 is often a flexible membrane and made of a relatively soft material. Through the adhesive layer, the label pad, the test pad and the sample pad are tightly combined at the superimposing position, and especially, sides of the sample receiving pad 104 and the label pad 103 are bonded onto the surface of the first support sheet 111, and the other sides of the sample receiving pad 104 and the label pad 103 are bonded together through the adhesive layer of the flexible covering layer, such that the sample receiving pad 104 and the label pad are formed integrally, helping the liquid flowing direction to be kept consistent. In some embodiments, one end of the flexible covering layer covers the part of the sample application pad 104, one end of the sample receiving pad 104 is allowed to expose (the part as numbered in 1032), to contact the liquid sample. In fact, when the test strip is inserted into the liquid sample, the exposed part 1032 of the sample receiving pad 104 is allowed to contact the liquid, the area not covered by the non-absorbent covering layer contacts the liquid or partially contacts the liquid and the liquid is allowed to flow from the sample pad 104. Especially, when the test strip is inserted into a container in advance, the liquid sample is added into the container; for example, the test strip is in advance provided in a urine cup, one end of the sample receiving pad 104 of the test strip is allowed to approach the bottom of the urine cup, and then urine is injected into the urine cup, but urine directly urinating into the urine cup will impact the test strip, causing a flood phenomenon. If only a part of the sample receiving pad 104 of the test strip is exposed (the part as numbered in 1032) and the other part thereof is covered by the first non-absorbent covering layer 105, no excess liquid impacts the sample receiving pad 104, which causes the liquid to be absorbed only by the sample pad. The hydrophobic medium is coated on the back face of the first support sheet 111 with the test strip, or the second support sheet 113 is bonded onto the back face of the first support sheet 111, thus preventing urine from flowing downstream along the test strip from the back face. Thus, the liquid can be absorbed only through the sample receiving pad 104 of the test strip, and can never flow to the downstream of the test strip from other places without the capillary force to wet some areas on the test strip in advance, such as the label pad, the test line or the control line on the test pad. Thus, test reliability is improved and especially sensitivity and specificity are improved. This is especially important for those who have no operating experience and professional skills in home testing.

Generally, the sample receiving pad 104 and the label pad 103 are fluffy in a natural state; especially when the sample receiving pad 104 is made of non-woven fabric and sponge, its shape and thickness are uneven, which directly affects the capillary force of the label pad and the flow velocity of the liquid on the sample receiving pad. If there are multiple test pads in a same batch, although the label pad and the sample receiving pad that have a same width are used in each test pad, the test pads have different fluffy states, some of the test pads have large thickness, and some of the test pads have small thickness. Therefore, the inconsistent thickness directly affects the flow velocity of the liquid thereon and diffusion uniformity. In addition, it also directly affects the dissolution of the label on the dry label pad by the liquid, this is due to a fact that different fluffy degrees of the test pads or the label pads actually indicate the distribution state of fibers, the distribution state of fibers directly affects the flow of the liquid, and after all, the flow of the liquid enables the capillary force of the fibers to be increased. When the flow velocity of the liquid is slow, the label may be slowly dissolved and a large amount of the label exists in the solution. When the flow velocity of the liquid is fast, the label may be fast dissolved and a small amount of the label exists in the solution. When the label reaches the testing area, the final testing results will be directly affected due to different amounts of the label in the solution.

Therefore, in a specific embodiment of the invention, the label pad and the sample receiving pad are covered with the rolling and stretching layer 106, 50 (as shown in FIG. 9A and FIG. 9B, FIG. 7, FIG. 4, FIG. 3B, and FIG. 1 and FIG. 2), and the rolling and stretching layer is rigid and has a specified weight; therefore, when the rolling and stretching layer covers the test pad or the label pad, a specified pressure can be given to the test pad or the label pad, thereby changing the fluffy state of the test pad, making the test pad uniform and reducing the error between different reagent strips. Here, a side of the rolling and stretching layer contacting the test pad is coated with the adhesive, and the rolling and stretching layer can be coated on the test pad and the label pad through the adhesive, such that the test pad and the label pad are smooth or uniform, and the flow velocity of the liquid on each test pad is kept substantially consistent; in addition, the test pad is superimposed on the label pad, the label pad is stably and tightly superimposed at two superimposing positions on the test pad, thus ensuring the continuity of liquid flow and the capillary force of liquid flow. The rolling and stretching layer here can be made of a same rigid material as the support sheet. The "rigidity" here is relative to the materials of the sample receiving pad and the label pad. Generally, the test pad and the label pad are made of flexible absorbent materials and can be compressed, while the rolling and stretching layer is made of a non-absorbent material, and has a relative weight relative to the sample pad or the label pad; for example, under the high density and the same thickness, a pressure can be applied to the surfaces of the sample receiving pad and the label pad, such that the thickness of the sample pad and the label pad is uniform, the flow velocity of the liquid on each test strip is consistent, and the dry label can be dissolved, overcoming the area and shape of the liquid flow as shown in FIG. 8B and FIG. 8C. For example, as shown in FIG. 4, the rolling and stretching layer 106 is divided into a plurality of areas 107, 108, 109 by a dividing line 901, and similarly divided into several areas by the dividing line. The dividing line is similar to a scribed line, by which the rolling and stretching layer is divided into a plurality of areas. Therefore, the rigid rolling and stretching layer can be folded. Due to the effect that the label pad and the sample pad are raised by the second support pad below them, in fact, when the test strip is assembled into the clamping slot of the carrier, the sample pad and the label pad of the test strip are in the form of a slope with an arc, such that the rolling and stretching layer 106 can also be turned into the arc due to the effect of the scribed line and bonded onto the label pad and the sample pad.

In some embodiments, the test strip may include one label pad or a plurality of label pads (as shown in FIG. 1 and FIG. 2). "Superimposed on each other" here means that a plurality of label pads are sequentially arranged on the test strip along the longitudinal direction of the test strip, for example, as shown in FIG. 1. The arrangement means that the label pads are superimposed end to end instead of being connected end to end. The so-called "superimposed" means that two label pads are superimposed and combined with each other and have a place where they are staggered. For example, as shown in FIG. 1, reference symbols 41 and 42 denote two different areas of one label pad 46, namely a label area 41 and a label-free area 42. Thus, other three label pads 43, 44, 45 with the same area each include the label area and the label-free area. Here, a first label pad 81 is located downstream of the sample pad 70, a second label pad 43 is located downstream of the first label pad 81, a third label pad 44 is located downstream of the second label pad 43, and a fourth label pad 421 is located downstream of the third label pad 44. In an embodiment, the superimposing mode is that the white area 42 of the first label pad 81 is located above the colored part of the second label pad 43, and the white area of the second label is located above the third label area, and such areas are superimposed together on each other. According to the mode, four label pads are superimposed on each other end to end to form the label pad area 40. Of course, when superimposed with the NC membrane 21, the white label-free area of the fourth label pad 45 is superimposed on one end 202 of the NC membrane, and they have a superimposing area 46.

In some embodiments, the length of one label pad is generally 6-8 mm, the length of the label-free area is generally 2-3 mm, and the length of the label area is 3-5 mm; for example, the length of the label-free area is 3 mm, the length of the label area is 5 mm, and the length of the area superimposed with the second label pad 43 is 1 mm. At least, a part of the label-free area of the first label pad, instead of the whole white label-free area, is superimposed with the label area of the second label pad 43 to cover the second label pad 43. According to the above principles, a plurality of label pads can be superimposed and connected together. In some embodiments, the label here includes colored particles and an antibody or antigen conjugate that specifically binds to the analyte. These labels can be moved by the liquid sample and flow to the downstream NC membrane, for example, flow through the testing area 202 and control area 203 of the NC membrane, and are finally absorbed by the absorption pad 30.

In some embodiments, as shown in FIG. 1 and FIG. 2, a flexible covering layer 80 covers the label area, where the flexible covering layer has a function similar to a strap, the "strap" here is similar to a fixing strap, one end of the strap is connected to the sample pad 70 and the other end thereof is connected to one end of the NC membrane to cover the label pads, such that the label pads are more tightly superimposed with each other, and the change in the mutual position of the sample pads is avoided. The flexible layer 80 has two faces, one of which faces the face of the label pad and the other of which faces outward. The face of the flexible layer 80 facing the label pad has the adhesive layer and is bonded onto each label pad, such that the superimposing areas of the label pads are more tightly bonded together, one end of the label pad covers the part of the sample pad and the other end thereof covers the NC membrane, and then the white label-free area of the fourth label pad is more tightly bonded onto one end 201 of the NC membrane.

In an embodiment, the partial area of the sample pad 70 covers the whole first label pad 46 and also covers the part of the label area of the part of the second label pad 43. In some embodiments, the flexible layer 80 is further covered with the rolling and stretching layer 50. Substantially, four areas formed by the dividing lines are bonded onto the flexible layer 80, such that four strips substantially cover the superimposing area of two label pads, and a plurality of label pads are more tightly combined together to form a complete test strip together with the NC membrane and the sample pad. The main function of the rolling and stretching layer here is to make a plurality of label pads stably stacked together.

In an embodiment, the labels on the label areas of the plurality of label pads are the same, and include colored particles and conjugated antibodies that specifically bind to the analyte in the sample. For example, the antibody can be an antibody of a small drug molecule, and when it is tested by the competitive method, the antigen of a small drug molecule is immobilized on the testing area. For example, during the test of THC, the label area is coated with the antibody that specifically binds to THC in the sample, the antibody is conjugated with the label of gold particles or latex particles, and the diameter of the gold particles or latex particles can be in millimeters or nanometers. However, the antigen corresponding to THC is immobilized on the testing area, for example, BSA is connected to THC molecules to become the antigen. Thus, when the sample includes THC, THC molecules in the sample are connected with the antibody on the label pad; and when they flow to the test line, the THC antigen immobilized on the test line competes with the THC molecules in the sample to bind the antibody conjugated with the label particles. The basic principle of the competitive method is also known technical content in the art, and will not be repeated again.

In addition, generally, the test strip only includes one label pad, and its length may be longer. However, the label pads of the invention are provided in plurality, and the blank area of the first label pad covers the label area of the second label pad, such that multiple labels are connected and covered in this way. The competitive method can improve the sensitivity and discrimination of test, especially for small drug molecule THC. In addition, the test strip can obviously have a gradient and is more easy to identify. Especially when the analyte in the sample is approximate to a test threshold, it is hoped that negative and positive testing results can be correctly distinguished. Therefore, when a threshold is selected, the distinguishing feature of the test strip is detected at the -50% cut off threshold and the +50% cut off threshold. If the test strips have different levels at the two thresholds and the color lines are obviously distinguished, this means that in actual detection, the lines are obviously distinguished and positive or negative samples can be easily distinguished. The distinguishing feature here is that a great difference in color level indicates the obvious distinguishing of the color lines. The negative result here does not mean that the sample includes no THC, but means that the content of the THC in the sample is lower than a specified threshold. After all, if the drugs are up to the drug abuse standards during the test of the drug abuse, the content of the analyte in the sample is relatively high. If the drugs are taken normally instead of being abused, the content of the analyte in the sample is relatively low, but this does not mean that the sample does not include the analyte. Therefore, the threshold is a changing value and unchanged for a period of time, and can also be stipulated according to the actual situations of different countries.

For example, when the test threshold of THC is 50 ng/ml, if THC is tested at -50% cut off quality control sample (25 ng/ml) and +50% cut off quality control sample (saliva, 75 ng/ml), and if there is a large difference between the line colors, it is easy to distinguish them in actual test, without causing false positive or false negative results. THC is a special small drug molecule and generally has low content in samples such as urine or saliva. At present, the test threshold is set at 50 ng/ml. When the content of THC in the sample is higher than the threshold, the testing result is positive; and when the content of THC in the sample is lower than the threshold, the testing result is negative. When the competitive method is adopted, the positive result shows absence of lines and the negative result shows presence of lines, the deep line indicates the low content of the sample or no sample.

### Testing device

In some embodiments, that test strips of the invention, as shown in FIG. 1 and FIG. 2, FIG. 4 - FIG. 7 and FIG. 9 can be carried by a carrier. Of course, the test strips not mentioned in the invention can also be carried by the carrier (as shown in FIG. 3B). These test strips carried by the carrier are generally arranged in a chamber, and the chamber is a container for directly collecting saliva and urine. When the saliva or urine is collected in the chamber, the urine directly contacts the test strips on the carrier, and the analytes in these liquid samples are tested or assayed.

Therefore, when the carrier is arranged in the chamber, a testing device is constituted. Generally, the chamber includes a bottom, side walls and an opening, and the opening is used for receiving the liquid sample. In some embodiments, the sample pad of the test strip on the carrier is proximal to the bottom of the chamber, while the carrier leans against or is attached to the side walls. When the liquid sample enters the chamber, the liquid sample at the bottom contacts the sample pad to assay the analyte.

In some embodiments, the carrier is in the form of a card, such as a plastic card, with a specified thickness, and a plurality of clamping slots are formed in the carrier and each carries a test strip, the depths of these clamping slots are consistent and generally consistent with or slightly greater than the thickness of the test strip. In some embodiments, as shown in FIG. 9 - FIG. 12, one end of the clamping slot 803 in the carrier 800 has a bottom 807, while the other end thereof is provided with an opening 808 and the bottom 8031 of the clamping slot. When the test strip 90 is arranged in the clamping slot, the part of the sample pad 905 of the test strip extends outward from the opening 808 of the clamping slot (as shown in FIG. 11, FIG. 12 and FIG. 10). However, other parts of the test strip, such as the part of the sample pad, the label pad, the test pad 902 and the absorbent pad 906, are located in the clamping slots, and the end of the absorbent pad contacts with the bottom 807. It can be understood that the part of the sample pad of the test strip is located in the clamping slot 803, and the other part thereof is exposed from the opening 808. Here, the bottom surface 8031 of the clamping slot is a flat surface. In the conventional way, a layer of flexible membranes covers the front face of the clamping slot 803 and these flexible films seal the clamping slot 803 to form a channel with one end being sealed and one end being open, and the test strip 90 is located in the channel, where the opening of the channel is the opening 808 of the channel formed by the clamping slot and the flexible membrane, so the opening 808 here can be the opening of the clamping slot; and after the opening of the clamping slot is covered with the membrane, the opening 808 of the channel is formed. In this case, when one end of the carrier 800 with the test strip is inserted into the liquid, part of the liquid enters the channel from the opening 808 due to the presence of the sealed channel. Due to the air pressure, the liquid will not enter the channel after entering the channel at a certain distance, and the part of the sample pad 905 of the test strip 90 absorbs the liquid sample to complete the test. In this case, the liquid entering the channel through the opening 808 may enter the clamping slot along the capillary gap formed between the back face of the test strip and the bottom surface 8031 of the clamping slot, while the back face of the conventional test strip does not have a hydrophobic layer (or the test strip improved in the invention is used and includes the hydrophobic layer or the second support sheet). In this case, the liquid will quickly flow into the clamping slot, thereby wetting the testing area 902 in advance and resulting in the failure of the test. If such a carrier is arranged in a chamber, the partially exposed sample pad 905 is proximal to the bottom of the chamber. If a large amount of liquid enters the chamber, the liquid has a relatively strong impact force relative to the test strip and may also flow into the channel through the opening 808, thereby wetting the downstream label pad or test pad in advance through the capillary gap formed by the back face of the test strip and the bottom surface 8031 of the clamping slot.

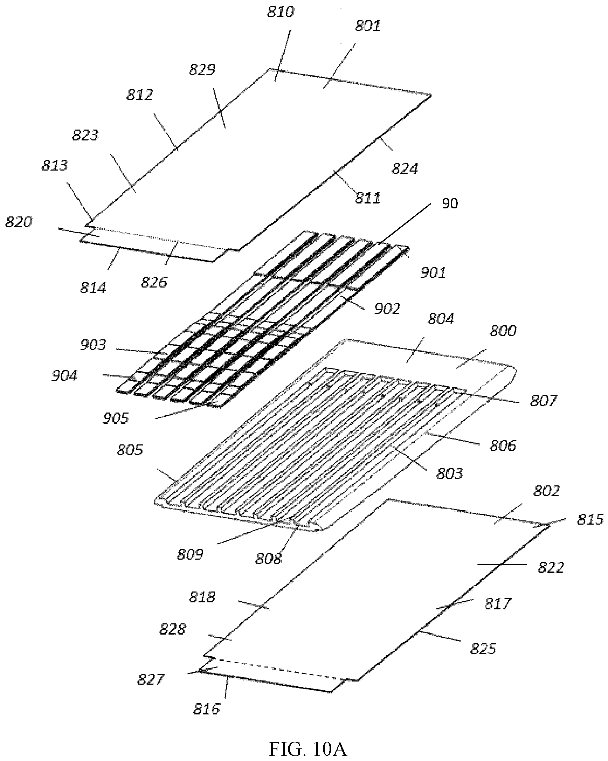

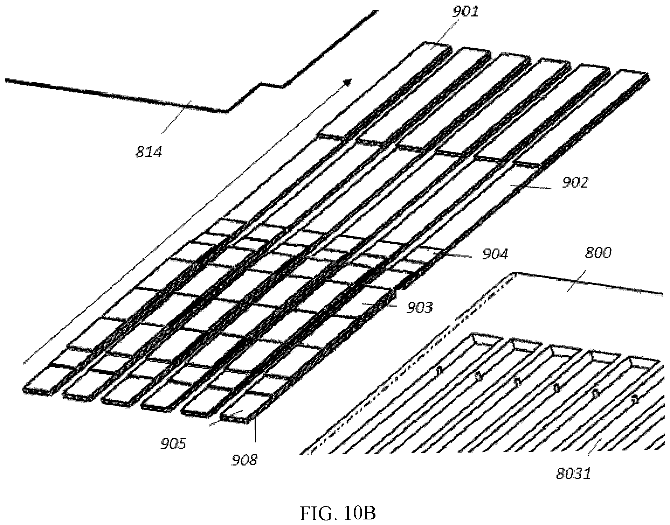

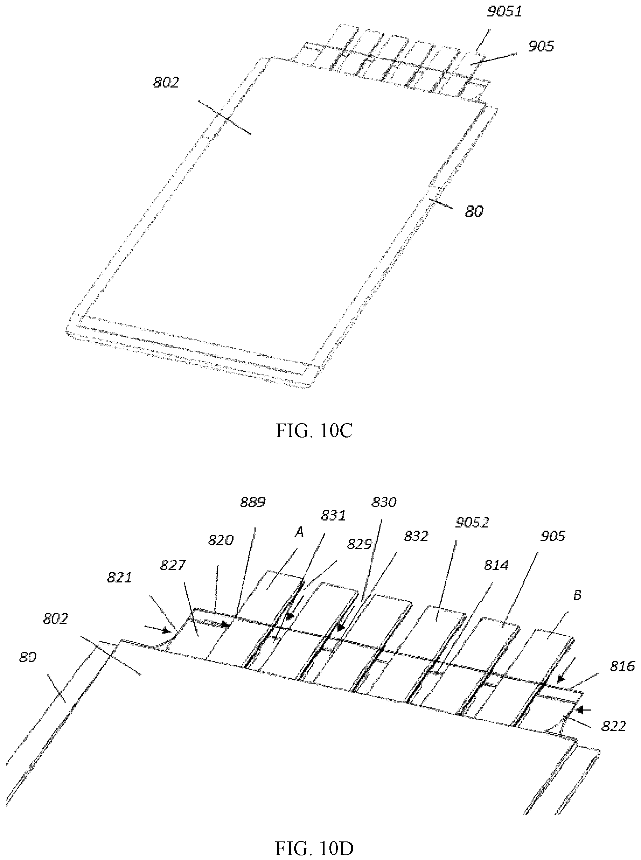

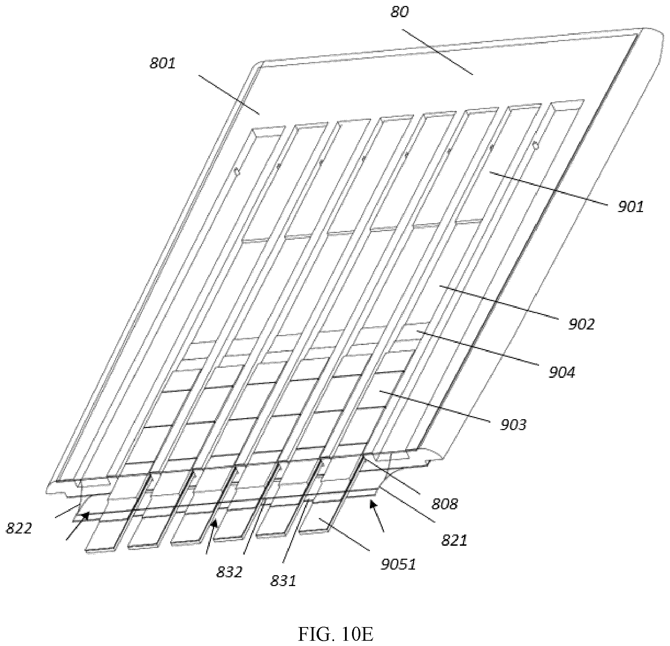

When common test strips are designed in the invention, they can prevent the liquid from directly entering the capillary gap formed between the back face of the test strip and the bottom surface 8031 of the clamping slot through the opening 808, and at least this phenomenon can be delayed. For example, a first covering membrane or a first covering layer 801 is provided; the first covering membrane has two ends, namely an end 810 and an opposite end 814. The covering membrane has two areas, where one area is a covering area 829 and the other area is a blocking area 820 and covers the exposed sample pad, while the covering area 826 directly covers the clamping slot to form a channel with one end being sealed and the other end being open. The blocking area can delay or reduce the chance that the liquid sample enters the channel through the opening 808 of the channel or hardly make the liquid enter the channel, and completely depend on the capillary force on the test strip to absorb the liquid and make the liquid flow on the blocking area. In an actual product, for the convenience of covering, the size of the covering layer 801 is equivalent to that of the carrier 800, and the transverse width of the covering layer is the same as that of the carrier 800, but the longitudinal length of the covering layer is greater than that of the carrier 800, and then the blocking area is formed. When covering is needed, the end 810 of the covering layer 801 covers an end 804 of the carrier 800, while two lateral sides 812, 811 respectively cover two sides 805, 806 of the carrier, and the covering area 829 covers the clamping slot, such that a large proportion of the test strip 90 is located in the channel formed by the clamping slot 803 and the covering area 829 of the covering layer 80, and the blocking area 820 covers the front face 9051 of a part of the exposed sample pad 905. Therefore, the end 814 of the covering layer contacts with the exposed sample pad 905. The function of the blocking area is that when a large amount of liquid flows into the carrier and contacts with the test strip, because the blocking area 820 covers the part of the sample pad 905, the liquid does not directly impact the test strip and the flow velocity of the liquid around the part of the sample pad 905 of the test strip will slow down. When the flow velocity of the liquid slows down, the liquid is not easy to flow into the capillary gap formed by the back face of the test strip 90 and the bottom surface 8031 of the clamping slot 803 through the opening 808 of the channel, so as to prevent the liquid from flowing into the testing area 902 in advance and wetting the testing area. In addition, the liquid is not easy to directly flow into the opening 808 of the clamping slot (at least when the flow velocity of the liquid is very low), because the blocking area delays the flow velocity of the liquid in advance, the sample pad 905 of the test strip still contacts the liquid sample, and the liquid sample can still flow on the test strip to complete the test. In a preferred embodiment, a covering layer 802 (second covering layer) is arranged on the back face of the carrier to cover the back face of the carrier 800, shares a size with the carrier 800, and also has two areas, namely, a covering area 828 and a blocking area 827, where the covering area covers the back face 9052 of the part of the sample pad 90 (as shown in FIG. 10), and the part of the sample pad 905 is also exposed from the opening 808 of the clamping slot 803. Each covering layer has two faces. The first covering layer 801 covers the front face of the carrier 800, and has a front face 823 and a back face 824. The adhesive layer is coated on the back face, so that it can cover the periphery of the clamping slot 803 to form a sealed channel. The adhesive layer is also provided on the back face of the blocking area 820, and bonded to the front face 9051 of the part of the sample pad 905 of the test strip 90. When the covering layer is flexible, it is tightly bonded onto the sample pad. In some embodiments, the second covering layer 802 also includes a first end 815 and a second end 816, two sides 818, 817, a front face 822 and a back face 825. During covering, the front face 822 is provided with the adhesive layer, such that the second covering layer covers and is bonded onto the back face of the carrier 800, and the second blocking area 827 is bonded onto the back face 9052 of the part of the sample pad 805. When the blocking area 820 of the first covering layer and the blocking area 827 of the second covering layer cover and are bonded onto the front face and back face of the part of the sample pad 905 respectively, as shown in FIG. 10 - FIG. 12, a relatively closed blocking area is formed at the sample pad 905, only the sample pad is exposed in the blocking area, and the back face of the test strip 90 and the opening 808 (sealed channel) of the clamping slot 803 are blocked not to directly contact the liquid sample. It is equivalent to provision of a protective fence outside the opening 808. The protective fence is formed by the flexible blocking areas of the first covering layer and the second covering layer, thereby reducing the liquid directly flowing to the opening 808. When the blocking area 820 of the first covering layer and the blocking area 827 of the second covering layer are flexible and have adhesive layers, the two blocking areas can be bonded together. For example, if a plurality of test strips are provided, gaps 829, 830 are obtained between the test strips. In the gap areas, the flexible blocking areas can be bonded together to form bonding points 831, 832, and these bonding points also prevent the liquid from entering the opening 808 through the spacing areas between the test strips. Thus, the sample pad 905 is guaranteed to contact the liquid sample to the maximum extent, and the liquid cannot directly flow into the opening 808 of the clamping slot 803 through the gaps 829, 830 between the test strips. In some embodiments, when the plurality of test strips are arranged in the plurality of clamping slots, two test strips are always distributed on the outermost side in a lateral direction, for example, the test strips A and B in FIG. 11 are distributed on two sides; and when the width of the blocking area 802 of the first covering layer and the width of the blocking area 827 of the second covering layer are greater than the width of the plurality of clamping slots (or the width of the plurality of test strips), bonding points 821, 8222 are formed on the lateral sides of the test strips A and B, and the liquid sample also cannot flow from sides of the test strip on the two sides into the opening of the clamping slot 803. It can be understood that when the clamping slots are provided in plurality, each clamping slot has a similar opening 808, such that the liquid sample can be prevented from directly flowing into the capillary gap formed between the back face of the test strip and the bottom surface 8031 of the clamping slot without directly flowing into the opening. This capillary gap is caused by mechanical errors and certainly exists. When the test strip and the clamping slot are assembled, it is substantially impossible to have no gap between the back face of the test strip and the bottom surface 8031 of the clamping slot, unless the back face of the test strip and the bottom surface 8031 of the clamping slot are bonded together by the adhesives. However, the operation is very complicated; due to the use of excessive adhesives, volatile substances will directly damage reagents such as antibodies and antigens on the test strip, and the operation also has the high requirements for the operating environment. In the invention, the blocking areas 820, 827 with two covering layers are skillfully bonded to each other to cover a part of the back face 9052 and the front face 9051 of the sample pad 905, and one blocking area is formed around the sample pad 905, such that the liquid sample is not easy to enter the opening 808 of the clamping slot. Generally, the covering layer is flexible, transparent or opaque, and the flexible blocking areas are bonded onto the front face and back face of the sample pad, and bonded between the spacing areas of the test strips and also bonded beside the outermost test strips. This bonding structure may, substantially in the case of even a large amount of liquid, make the liquid rarely flow into the opening 808 of the clamping slot at the beginning. However, the exposed sample pad 905 is partially covered by the flexible blocking areas instead of being completely covered by the flexible blocking areas, and the uncovered part is used to absorb liquid samples for testing and assaying.

Of course, it can be understood that a way to cover the carrier by the covering layer is adopted, regardless of the structure of the test strip (which may be the common structure of the test strip), for example, only the sample pad, the label pad and the test pad are bonded onto the first support sheet. Of course, the improved structure of the test strip provided in the invention can also be adopted. For example, the test strips as shown in FIG. 1 and FIG. 2 and FIG. 4 - FIG. 7 can also be carried on the carrier of the invention. Especially, when the test strips as shown in FIG. 1 and FIG. 2 are provided in the clamping slot 803 of the carrier 800 provided in the invention, because the plurality of label pads are provided, the label pads 81 superimposed with the sample pad 70 are proximal to the opening 808 in the clamping slot when the plurality of label pads are often superimposed; and if the liquid sample is allowed to directly flow into the opening 808, the liquid sample flows into the clamping slot to wet the label pad 81 in advance. However, the liquid flowing from the sample pad 70 cannot flow at a place where the label pad 81 gets wet in advance, resulting in the slow flow velocity of the liquid or the poor dissolution of the label, thus affecting the final testing results, such as inaccurate testing results. As the covering layer of the invention is designed, the liquid sample is not easy to flow to the opening 808, which can prevent the liquid from wetting the label area in advance and improve the accuracy of the test.

### Embodiments

The following further elaborates the invention with reference to accompanying drawings and examples. It should be noted that the embodiments are only a detailed description of the invention and cannot be used to limit the protection scope of the invention. All features disclosed in the embodiments of the invention or all steps of methods or processes disclosed therein can be combined in any way except mutually exclusive features and/or steps, and are within the protection scope of the invention. All technologies not involved therein can be achieved by existing technologies.

### Embodiment 1 Test strip

As shown in FIG. 1 and FIG. 2, a test strip includes a test strip main body 10, where the test strip main body 10 is made of a PVC material as a support pad, and a sample pad 70, a plurality of label pads 40, an NC membrane 20 and a water absorption end 30 are sequentially arranged on the support pad. The sample pad is used to apply a liquid sample, the label pad 40 includes four layers of colloidal pads or four colloidal pads, which can also be referred to the label pad herein. Each label pad 40 is made from colloidal gold applied (or mechanically sprayed) to non-woven fabric (or glass fiber), and is divided into a colored part 41 and a white part 42, where the colored part 41 is an area onto which the colloidal gold is applied, and the edge part 42 is an area onto which the colloidal gold is not applied.

The colored part herein is generally an area where the label is sprayed or coated, the label may be colored particles, and the colored particles may be latex or gold particles. The method of specific treatment in colored parts is the existing disclosed technology and will not be elaborated here. In some embodiments, the colored part is about 2 times, 3 times or 4 times as large as the white part.

Each label pad 40 is stacked together in a straight line, and the white part 42 of an adjacent label pad is stacked on the colored part 41 of another label pad. The length of the white part 42 is generally 2-6 mm, and in the embodiment, the length is 2 mm and the length of the colored part of colloidal gold is 6 mm. The length of the superimposing area between the white part 42 of one label pad and the colored part 41 of the adjacent label pad is less than 6 mm, and the length in the embodiment is 4 mm. Further, the colored part 41 of the label pad is superimposed with the colored part 41 of the adjacent label pad, and the superimposing length thereof is not more than 6 mm, and the preferred superimposing length thereof is not more than 5 mm, for example, it can be 1 mm, 2 mm, 3 mm or 4 mm. The NC membrane 20 is a nitrocellulose membrane, the upper end of the NC membrane contacts with the white part 42 of the nearest label pad 40, and the lower end thereof contacts with the water absorption end 30. Specifically, in the embodiment, four label pads are provided, and each label pad includes an area 41 sprayed with the label and an area 42 not sprayed with the label. For example, the first label pad, the second label pad, the third label pad and the fourth label pad are provided from upstream to downstream. The white part of the first label pad is superimposed on the colored part of the second label pad, and the superimposing length is 2 mm. The second label pad and the third label pad share the superimposing mode with the first label pad and the second label pad, and the third label pad shares the superimposing mode with the fourth label pad. On the last label pad, a white part 401 is superimposed on one end 201 of the test pad. The testing area in the embodiment is the NC membrane, and the testing area 202 and the testing result control area 201 are provided on the membrane. One end 31 of the absorption pad 30 is superimposed on the other end 21 of the NC membrane.

The test strip includes a flexible layer 80. The flexible layer 80 may have an adhesive property on a side thereof, one end of the flexible layer 80 partially covers a sample section 70, and the other end thereof is connected to one end of the NC membrane. The sample section 70 is made of non-woven fabric or glass fiber, bonded onto the test strip main body 10 by the adhesive, and used as a sample application area. The flexible layer 80 has the adhesive properties of the self-adhesive section 60 and is bonded onto the NC membrane. The flexible layer 80 completely covers the four label pads 40, such that the label pads 40 are more tightly superimposed together to prevent mutual position change. Four rolling and stretching layers 50 cover the flexible layer 80, and are PVC (polyvinyl chloride) strips or PS (polystyrene) strips and bonded together to each other to further fix the label pads, such that the label pads 40 are more tightly superimposed.

In addition, the water absorption end 30 of the test strip is made of an absorbent material, where the absorbent material can be absorbent filter paper, absorbent sponge, or the like. The upper end of the water absorption end 30 contacts with the NC membrane, and the lower end thereof is flush with the lower end of the test strip main body 10.

The upper end and the lower end are based on the length direction of the test strip main body 10. The direction of the test strip main body 10 near the water absorption end 30 is downstream, and the direction of the test strip main body 10 near the sample section 70 is upstream. Generally, the liquid flows from upstream to downstream, the NC membrane is located downstream of the label pad, and the label pad is generally located downstream of the sample pad.

### Embodiment 2 Sensitivity comparison experiment

(1) THC (tetrahydrocannabinol) test products from the competitive method are selected and assembled according to the available technology to make into THC test strips with one layer of label pads (before improvement) and THC test strips with five layers of label pads (after improvement) as described in Embodiment 1. The NC membrane and the sample pad are made of a same material, on which a same reagent is treated. The only difference is that labels with the same composition (before improvement) are sprayed on one label pad, and that the labels with the same composition (after improvement) are respectively sprayed on five label pads on average.

The test strips before and after improvement are used to respectively test negative samples (NEG) (excluding THC), -50% cut off quality control samples (25 ng/ml) and +50% cut off quality control samples (saliva) (75 ng/ml), and the cut off value of THC is 50 ng/ml. Ten clinical samples (saliva samples, which are determined as negative samples by chromatography) from real human are selected and observed for signal intensity. The signal intensity is compared with the colourimetric card (FIG. 3) and the testing results are recorded and shown in Table 1 below. The colourimetric card is numbered G1 to G10 according to the color from light to dark, the great value of G indicates a strong signal and low content of THC, and the small value of G indicates a weak signal and high content of THC.

**Table 1 Comparison of testing results of THC test strips before and after improvement**

| Sample name | | Testing result | |
|---|---|---|---|
| | | Before improvement | After improvement |
| Quality | NEG (negative) | G5 | G7 |
| control | -50% cutoff | G4.5 | G6 |
| sample | +50% cutoff | G4 | G3 |
| Clinical sample | 1 | G5 | G8.5 |
| | 2 | G5 | G8.5 |
| | 3 | G6 | G8.5 |
| | 4 | G7 | G8 |
| | 5 | G5 | G8.5 |
| | 6 | G6.5 | G8.5 |
| | 7 | G5 | G8 |
| | 8 | G6 | G8.5 |
| | 9 | G6.5 | G8.5 |
| | 10 | G6 | G9 |

From the results in Table 1, it can be seen that the signal measured by THC test strips with five layers of label pads (after improvement) is stronger, the color of -50% cut off quality control sample is G6 and the color of +50% cut off quality control sample is G3, and the range of the testing results is wider, with a difference of 3 color levels. It shows that the THC test strips with four layers of label pads (after improvement) have higher sensitivity and specificity, make the testing results more reliable and can also be used to detect samples with lower concentration. However, if one label pad is provided, the color of the corresponding 50% cut off quality control sample is G4.5 and the color of the +50% cut off quality control sample is G4, with a difference of 0.5 color level. Thus, when the color level is approximate to a detection threshold, it is difficult to directly distinguish between negative and positive, which will lead to false positive or false negative.

(2) HCG (human chorionic gonadotropin) test products from the sandwich method are selected and assembled according to the available technology to make into HCG test strips with one layer of label pads (before improvement) and HCG test strips with four layers of label pads (after improvement) as described in Embodiment 1. 10 miU/ML quality control sample, 25 miU/ML quality control sample, 100 miU/ML quality control sample, and 500 miU/ML quality control sample are respectively tested by the test strips before and after improvement; and 10 clinical negative samples and 10 pregnancy samples from real human are respectively tested by the test strips before and after improvement and observed for signal intensity. The signal intensity is compared with the colourimetric card and the testing results are recorded and shown in Table 2 below. The colourimetric card is numbered G1 to G10 according to the color from light to dark, the great value of G indicates a strong signal and low content of HCG, and the small value of G indicates a weak signal and low content of HCG.

**Table 2 Comparison of testing results of HCG test strips before and after improvement**

| Sample name | | Testing result | |
|---|---|---|---|
| | | Before improvement | After improvement |
| Quality control sample | 10 miU/ML | G3 | G4 |
| | 25 miU/ML | G4 | G5 |
| | 100 miU/ML | G6 | G7 |
| | 500 miU/ML | G8 | G9 |
| Clinical negative sample | 1 | G1 | G1 |
| | 2 | G1 | G1 |
| | 3 | G2 | G1 |
| | 4 | G1 | G1 |
| | 5 | G1 | G1 |
| | 6 | G1 | G1 |
| | 7 | G2 | G1 |
| | 8 | G1 | G1 |
| | 9 | G1 | G1 |
| | 10 | G2 | G1 |
| Clinical pregnancy (positive) sample | 1 | G9 | G9 |
| | 2 | G9 | G9 |
| | 3 | G8 | G9 |
| | 4 | G10 | G10 |
| | 5 | G7 | G7 |
| | 6 | G8 | G8 |
| | 7 | G8.5 | G9 |
| | 8 | G8 | G9 |
| | 9 | G9 | G9 |
| | 10 | G9 | G9 |

From the results in Table 2, it can be seen that the signal measured by HCG test strips with four layers of label pads (after improvement) is stronger, the signal of clinical negative samples is lower, and the signal of positive quality control samples is stronger. The results show that compared with the HCG test strip with one layer of label pads, the HCG test strip with four layers of label pads (after improvement) has higher sensitivity and specificity, makes the testing results more reliable and can detect HCG samples with lower concentration.

### Embodiment 2

Experiment 2-1: such a testing device as shown in FIG. 10A - FIG. 10E is used, six test strips are provided on the carrier and used to test the analyte of HCG, front and back membranes cover a part of the exposed sample pad, the test strips between each sample pad are bonded together by the front and back membranes to form similar bonding points 831, 830, and the test strips at two sides are bonded together through the front and back membranes to form bonding points 822, 821, where the test strips are ones as shown in FIG. 9A and FIG. 9B.

Experiment 2-2: the structure of the carrier is the same as that in Experiment 2-1, the conventional test strip is adopted, and there are no second support sheet 113 and the flexible layer 112 as shown in FIG. 9A and FIG. 9B.

Experiment 2-3: the structure of the carrier is the same as that in Experiment 2-1. The test strip as shown in FIG. 7 is adopted, and a layer of hydrophobic adhesive is coated on the back face of the first support sheet 111.

Experiment 2-4: the carrier of the testing device here is still the carrier 800 as shown in FIG. 10A, but the covering membrane has no a blocking area 820 and actually only has a covering area. The covering area covers clamping slots 803 and a partition strip 809 of each of the clamping slots 803. The length of the covering area is equivalent to the length of the clamping slot, and only a sealed channel is formed, but the blocking area is not provided. The conventional test strip is adopted, and there are no the second support sheet 113 and the flexible layer 112 as shown in FIG. 9A and FIG. 9B.

Then, 20 testing devices are made in each experiment, each testing device has 6 test strips, and each test strip is used to test the analyte of THC. Then, specific quality control samples are four quality control urine including negative urine (NEG) (excluding HCG), 25 miU/ML quality control urine, 100 miU/ML quality control urine and 500 miU/ML quality control urine. Each sample is contained in 80 glass containers, and the height of urine in each container is 20 cm. When one end of the carrier exposed out of the sample pad is inserted into the container, the liquid can be higher than the position of the opening 808 of the channel for testing. Whether the T line of the test pad is tested successfully is determined, each sample is tested for 20 times, and the number of T lines successfully and unsuccessfully tested is counted.

The results are recorded as follows:

| Treatments | Negative urine (20) | 25 miU/ML | 50 miU/ML | 100 miU/ML |
|---|---|---|---|---|
| Experiment 2-1 | G1/20 | G5/20 | G7/20 | G9/20 |
| Experiment 2-2 | G1/18, 2 unsuccessful tests | G5/17, 3 unsuccessful tests | G7/18, 2 unsuccessful tests | G9/19, 1 unsuccessful test |
| Experiment 2-3 | G1/17, 3 unsuccessful tests | G5/16, 4 unsuccessful tests | G7/17, 3 unsuccessful tests | G9/17, 3 unsuccessful tests |
| Experiment 2-4 | G1/14, 6 unsuccessful tests | G5/13, 7 unsuccessful tests | G7/14, 6 unsuccessful tests | G9/14, 6 unsuccessful tests |

It can be seen from the above results that when the liquid directly flows from another place without passing through the sample pad, the label pad and the test pad on the test strip, the upstream area of the T line of the test pad gets wet in advance. In terms of the number of unsuccessful tests, the invention can effectively avoid the flow direction of the liquid, reduce the problem of wetting the test pad in advance and the failure rates in Experiments 2-1, 2-2 and 2-3 to varying degrees, and prevent the liquid from directly entering the opening 808. Even if some of the liquid enters the opening, the liquid is prevented from wetting the test pad in advance due to the existence of the second support sheet and the hydrophobic layer. However, for the conventional device, such as Experiment 2-4, because the liquid can directly enter the opening 808, the liquid can wet the test pad in advance, resulting in a high test failure rate.

All the patents and publications mentioned in the description of the invention indicate that these are public technologies in the art and can be used by the invention. All the patents and publications cited here are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of" and "consisting of" in each example here may be replaced by the rest 2 terms. The term "a/an" here merely means "one", but does not exclude including 2 or more instead of including only one. The terms and expressions which have been employed here are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. A test strip for testing an analyte in a liquid sample, comprising a first support sheet, wherein a sample pad, a label pad and a test pad are sequentially arranged on a front face of the first support sheet from upstream to downstream; the label pad comprises a label that is capable of flowing with a liquid; the test pad comprises a fixed binding substance that is incapable of flowing with the liquid; and a layer of hydrophobic medium is coated on a back face of the first support sheet, to prevent the liquid sample from flowing from the back face of the first support sheet and wetting the test pad or the label pad in a downstream area in advance when the sample pad contacts the liquid sample.

2. The test strip according to claim 1, wherein the hydrophobic medium is a hydrophobic adhesive.

3. The test strip according to any one of claims 1-2, wherein the hydrophobic adhesive comprises a polyurethane adhesive, a fluororubber adhesive or a siloxane adhesive.

4. The test strip according to any one of claims 2-3, wherein a second support sheet is bonded onto the hydrophobic adhesive.

5. The test strip according to claim 4, wherein the second support sheet is located below the sample pad and/or the label pad.

6. The test strip according to any one of claims 4-5, wherein the second support sheet is located below the sample pad and the label pad.

7. The test strip according to any one of claims 4-6, wherein the second support sheet comprises two ends, a first end is located below the label pad, and a second end is located below a superimposing position of the label pad and the test pad.

8. The test strip according to claim 7, wherein the first end of the second support sheet is shorter than an end of the sample pad.

9. The test strip according to any one of claims 4-8, wherein a back face of the second support sheet also comprises a layer of hydrophobic adhesive.

10. The test strip according to any one of claims 4-9, wherein the first support sheet and the second support sheet are made of a same material and are both non-absorbent.

11. The test strip according to any one of claims 4-10, wherein a flexible layer is bonded to the back face of the second support sheet, a length of the flexible layer is the same as that of the first support sheet, and the flexible layer is non-absorbent.

12. The test strip according to any one of claims 1-11, wherein the label pad comprises two or a plurality of label pads, and each label pad comprises the label.

13. The test strip according to claim 12, wherein each of the label pads comprises the same label.

14. The test strip according to claim 13, wherein plurality of label pads are stacked together end to end in a straight line, and a white part of one label pad of the plurality of label pads is partially superimposed on a colored part of another adjacent label pad.

15. The test strip according to claim 14, wherein the label pad comprises 3-8 label pads.
